# EUROPEAN PATENT SPECIFICATION

(11) **EP 1 767 211 B1**
(45) Date of publication and mention of the grant of the patent: **04.04.2012**
(21) Application number: 06020126.6
(22) Date of filing: 26.09.2006
(51) Int. Cl.: A61K 31/728, A61P 25/00, A61P 37/00

(54) **Pharmaceutical agent containing hyaluronan as an active ingredient**
Pharmazeutische Zusammensetzung Hyaluronan als aktive Komponente enthaltend
Composition pharmaceutique comprenant du hyaluronan comme ingrédient actif

(30) Priority: 26.09.2005 JP 2005277918; 12.10.2005 JP 2005297991; 01.12.2005 JP 2005348017
(43) Date of publication of application: 28.03.2007
(73) Proprietor: Glycoscience Laboratories, Inc., Tokyo, 113-8549 (JP); SHISEIDO Co., Ltd., Chuo-ku, Tokyo 104-8010 (JP)
(72) Inventor: Asari, Akira, c/o Gycoscience Lab., Inc., Yushima, Bunkyo-ku, Tokyo, 113-8549 (JP); Yamanokuchi, Hiroko, c/o Gycoscience Lab., Inc., Yushima, Bunkyo-ku, Tokyo, 113-8549 (JP); Kato, Tadahiko, c/o Glycoscience Lab., Inc., Musashimurayama-shi, Tokyo 208-0011 (JP)
(74) Representative: Brosch, Oliver

(56) References cited:
- EP-A- 1 166 788
- EP-A1- 1 300 412
- EP-A2- 1 034 780
- WO-A-97/11710
- WO-A-2005/049047
- WO-A-2005/067944
- CA-A1- 2 519 797
- JP-A- 9 227 386
- US-A- 6 013 641
- US-A1- 2004 071 740
- US-B1- 6 537 968

## Description

### Field of the Invention

The present invention relates to a tetrasaccharide according to claim 1 for use in the treatment or preventive of a multiple sclerosis via intraspinal administration.

### Background of the Invention

Hyaluronan is a long chain polysaccharide constructed from disaccharide repeating units each consisting of a D-glucuronic acid and an N-acetyl-D-glucosamine, and its oligosaccharide form is also known. Hyaluronan is an extract from a biological tissue, such as a rooster comb, umbilical cord, skin, and articular fluid, or is produced by a fermentative method using a Streptococcal bacterium. Since toxicological and immunological effects are not present, hyaluronan is utilized in a pharmaceutical or cosmetic component, such as in a well-known treatment of an arthritis employing an intraarticular injection of hyaluronan. In the following description, tetrasaccharide hyaluronan is designated as HA4.

HA4 was reported to have a therapeutic and inhibitory effect in an organ preservation, hepatic disorder and gastric ulcer (see, WO2002/004471). HA4 is also known to have a stress protein expression enhancing effect and a cell death inhibiting effect (see, Xu H, Ito T, Tawada A, Maeda H, Yamanokuchi H, Isahara K, Yoshida K, Uchiyama Y, Asari A. Effect of hyaluronan oligosaccharides on the expression of heat shock protein 72, J Biol . Chem. 2002, 10; 277 (19) : 17308-14). In addition, hyaluronan oligosaccharide was reported to have a variety of physiological activities (see, Asari A, Novel Functions of Hyaluronan Oligosaccharides. In Science of Hyaluronan Today, Editors: Vincent C. Hascall. Masaki Yanagishita Glycoforum, (http://www.glycoforum.gr.jp/science/hyaluroRan/HA.12/HA12J.html). 2005).

A multiple sclerosis is developed frequently during a period from adolescence to forties and accompanied with symptoms such as an unsteady walking, dim vision, double vision, difficulty in urination, and pain and numbness. When developed in pediatric or juvenile cases, it is sometimes accompanied with epilepsy. One or more pathologic foci responsible for the symptoms are developed diffusively in a cerebrum or spinal cord. Moreover, the pathologic foci are diffusive not only in terms of spatial diffusiveness but also in terms of temporal diffusiveness with occasional occurrence and disappearance. The pathologic condition of the multiple sclerosis involves an immune system, and is considered to be an autoimmune disease or inflammation. Also since the spinal cord nerve is injured, it is also considered to be one of a neural disease .

Cell viability means an active condition of a cell. Since some diseases involve reduced cell viability or cellular denaturation, an improvement in the cell viability is expected to provide a therapeutic effect in such a disease. The cell viability can be determined based on a Rhodamine 123 staining performance as an index. While a mitochondria acts pivotally in an energy metabolism, the fluorescent intensity of the Rhodamine 123 increases in a mitochondrial membrane potential-dependent manner. Accordingly, the Rhodamine 123 staining performance serves as an index of the mitochondrial activity, thus, an index of the cellular activity degree (see, Kim, M, Cooper DD, Hayes SF, Spangrude GJ, Rhodamine-123 staining in hematopoietic stem cells of young mice indicates mitochondrial action on rather than dye efflux. Blood, 1998 Jun 191(11): 4106-17).

A cytokine is a generic name covering proteinous factors (mostly glycoproteins), which are released from a cell and then mediate intercellular interactions such as immune or inflammatory reaction controlling effects, anti-viral effects, anti-tumor effects, and cellular growth/differentiation regulating effects. Those known as such cytokines include interleukins, interferons, tumor necrosis factors (TNF) and the like. On the other hand, chemokines are defined as a group of chemtactic cytokines having leukocyte chemotactic ability. As used herein, the chemokines are defined as a concept excluded from the cytokines.

A cytokine-associated gene refers generally to a gene which encodes the cytokine and a gene which regulates the expression of said gene. A variety of cytokine-associated genes are known, and a relationship between the promotion of a cytokine-associated gene expression and a disease is suggested. An effective inhibition of a cytokine-associated gene expression contributes greatly to the treatment of a variety of diseases.

EP-A-1300412 discloses hyaluronic acid as active ingredients of a heat shock protein (Hsp) expression promoter.

EP-A-1166788 discloses a complex of hyaluronic acid and zinc. US2004/071740A1 discloses hyaluronic acid for treating asthma. WO2005/067944A discloses hyaluronic acid for treating inflammatory disorders.

WO2005/049047A discloses a complex of hyaluronic acid and zinc for treating multiple sclerosis.

US6,537,968B1 discloses a composition of a protease inhibitor and hyaluronic acid as a penetrating agent for treating lupus erythematous.

JP09-227386-A discloses hyaluronic acid for preventing cell death. WO97/11710A discloses hyaluronic acid for treating autoimmune diseases.

US6,013,641-A discloses hyaluronic acid for treating graft-vs-host-diesease.

EP-A-1034780 discloses hyaluronic acid for treating psoriasis, dermatosis and allergic deratitis.

### Summary of the Invention

An obj ect of the present invention is to provide an autoimmune disease-treating agent, inflammatory disease-treating agent and neural disease-treating agent. Another object of the invention is to provide an autoimmune disease-preventing agent, inflammatory disease-preventing agent and neural disease-preventing agent.

Another object of the invention is to provide a novel cell viability enhancer.

Accordingly, an object of the invention is to provide a novel cytokine-associated genes and chemokine-associated genes expression inhibitor.

An autoimmune disease-treating agent, inflammatory disease-treating agent and neural disease-treating agent which accomplished the objects described above may contain hyaluronan as an active ingredient. Similarly, an autoimmune disease-preventing agent, inflammatory disease-preventing agent and neural disease-preventing agent which accomplished the objects described above may contain hyaluronan as an active ingredient.

A cell viability enhancer a which accomplished the objects described above may contains hyaluronan as an active ingredient.

A cytokine-associated gene and chemokine-associated gene expression inhibitor which accomplished the objects described above may contain hyaluronan as an active ingredient. Hyaluronan may have a function to inhibit the expression of cytokine-associated genes and chemokine-associated genes.

Thus, a cytokine-associated gene and chemokine-associated gene expression inhibitor may contain hyaluronan as an active ingredient. Hyaluronan employed herein is a tetrasaccharide containing 2 units, with a single unit being -D-glucuronic acid-β-1,3-D-N-acetylglucosamine-β-1,4-. Especially, it can inhibit the expression of pro-inflammatory cytokine-associated genes as cytokine-associated genes described above.

Since each of a pharmaceutical agent, cell viability enhancer, cytokine-associated gene and chemokine-associated gene expression inhibitor, may contain hyaluronan as an active ingredient, it can advantageously be produced readily at a large scale at a relatively low cost. Also since hyaluronan has almost no toxicity or antigenicity and enhances a therapeutic and prophylactic ability against disease which is possessed naturally by a living body of to prevent, it is expected to provide a therapeutic, prophylactic and inhibitory agent having an extremely reduced side effect. Thus, a pharmaceutical agent which is effective against an autoimmune disease, inflammation and neural disease can be provided. In addition, a pharmaceutical agent which is effective in the treatment of a disease attributable to a reduced cellular activity can be provided. pharmaceutical agent which is effective in the treatment of a disease attributable to a promotion of the expression of cytokine-associated genes and chemokine-associated genes.

### Brief Description of the Drawings

FIG. 2 shows a graph obtained by plotting the average of scores of EAE neural symptoms observed in multiple sclerosis model animals receiving a treatment with HA4 right after an inoculation on the ordinate and the days after the Day 0 inoculation on the abscissa.
FIG. 3 shows a graph obtained by plotting the average of scores of EAE neural symptoms observed in multiple sclerosis model animals treated with HA4 for 11 days after the onset of a disease on the ordinate and the days after the Day 0 inoculation on the abscissa.
FIG. 4 shows a graph obtained by plotting the average of a score of EAE neural symptoms observed in multiple sclerosis model animals treated once with HA4 immediately after inoculation on the ordinate and the days after inoculation on the abscissa.
FIG. 5 is a photograph of cells in each group prepared in Example 2.
FIG. 6 shows a performance representing results of a measurement of a fluorescent intensity in cells in each group prepared in Example 2.
FIG. 12 is a graph showing the results of a measurement of a production of an IL-1α and an IL-1β using a cytokine array,
FIG. 13 is a graph showing the results of a measurement of a production of an IL-6 and a TGF-β1 using a cytokine array.
FIG. 14 is a graph showing the results of a measurement of a production of a TNF-α and a TNF-β using a cytokine array.
FIG. 15 is a graph showing the results of a measurement of a production of an IL-6 using an ELISA.
FIG. 16 shows a schematic view representing an assumed action mechanism of HA4 in a treatment of a multiple sclerosis.

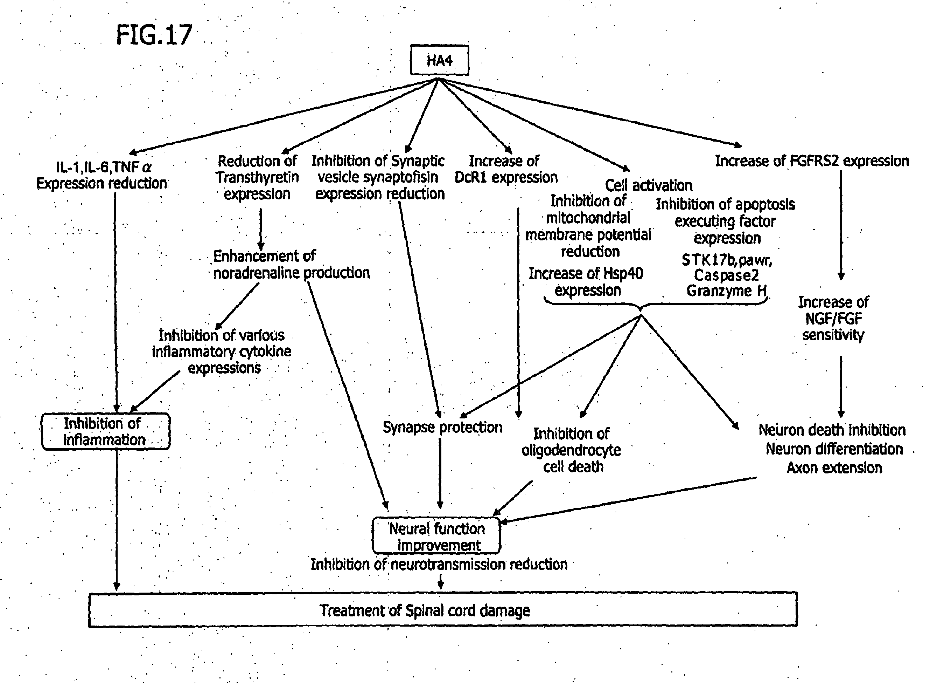

FIG. 17 shows a schematic view representing an assumed action mechanism of HA4 in a treatment of spinal cord injury.
FIG. 18 shows a schematic view representing an assumed action mechanism of HA4 against asthma and allergic disease.

### Detailed Description of the Preferred Embodiments

The invention is further detailed below. A cell viability enhancer is a pharmaceutical agent having a function of improving cellular viability. The term "improving cell viability" means that the cellular physiological viability is facilitated or that the reduction in the cell physiological viability is inhibited. An improvement in the cell viability results in a treatment or amelioration of a disease which reduces the cellular activity or a disease which causes a denaturation of the cell.

Cytokine-associated genes and chemokine-associated genes expression inhibitor agents having a function of inhibiting the expression of a variety of cytokine-associated genes and chemokine-associated genes. The phrase "inhibiting the expression of cytokine-associated genes and chemokine-associated genes" means that when comparing the expression level of relevant genes in an untreated animal cell with the expression level of relevant genes in an animal cell which has been treated with an agent, the latter is lower significantly. The expression level can be measured using a DNA chip formed by immobilizing a large number of probe DNAs on a substrate.

The invention is detailed below. In the following description, therapeutic and prophylactic agents, cell viability enhancer, cytokine-associated gene and chemokine-associated gene expression inhibitors, are referred collectively to as pharmaceutical agents.

Hyaluronan contained in a pharmaceutical agent is a tetrasaccharide according to claim 1 which includes two disaccharide units in which the position 1 of a β-D-glucuronic acid is bound to the position 3 of a β-D-N-acetyl-glucosamine and which is constructed basically from a β-D-glucuronic acid and a β-D-N-acetylglucosamine . Hyaluronan employed in the invention may typically be one extracted from a naturally-occurring material such as an animal, one obtained by a microorganism fermentation, one synthesized chemically or enzymatically. For example, hyaluronan can be obtained from a biological tissue, such as a crest, umbilical cord, skin, and articular fluid by an extraction method and a purification method known in the art. In addition, it can be produced also by a fermentative method using a Streptococcal bacterium.

It is preferred to produce hyaluronan i.e. the tetrasaccharide according to claim 1, having a low molecular weight specifically by reducing the molecular weight of the hyaluronan using a known method such as an enzymatic degradation, an alkaline degradation, a heat treatment, and an ultrasonication (Biochem. 33 (1994) p6503-6507), or by synthesizing chemically or enzymatically (Glycoconjugate J., (1993) p435-439, WO93/20827). For example, such an enzymatic degradation may be a method in which an enzyme capable of degrading the hyaluronan such as hyaluronan degradation enzyme (hyaluronidase (derived from testes), hyaluronidase (derived from Streptomyces), hyaluronidase SD and the like), chondroitinaseAC, chondroitinase ACII, chondroitinase ACIII, and chondroitinase ABC is allowed to act on the hyaluronan to yield hyaluronan oligosaccharide (see, Shin-Seikagaku Jikkenkoza, "Saccharides II-Proteoglycans and glycosaminoglycans", p244-248, Published in 1991, Tokyo Kagaku Dozin Co., LTD).

An alkaline degradation method may for example be a process in which a base such as an about 1N sodium hydroxide is added to an aqueous solution of hyaluronan which is then warmed for a several hours to reduce the molecular weight, and then an acid such as hydrochloric acid is added for neutralization whereby obtaining low molecular weight hyaluronan. hyaluronan employed in the invention i.e. the tetrasaccharide according to claim 1 includes its salt form, and a pharmaceutically acceptable salt can be employed as desired in view of the drug formulation. For example, it may be an alkaline metal salt, such as a sodium salt and potassium salt, an alkaline earth metal salt, such as a calcium salt and magnesium salt, an amine salt such as a tri (n-butyl) amine salt, triethylamine salt, pyridine salt, and amino acid salt.

A pharmaceutical agent of the present invention may be the hyaluronan, i.e. the tetrasaccharide according to claim 1. The pharmaceutical agent contains hyaluronan i.e. the tetrasaccharide according to claim 1 as an active ingredient, and can ameliorate one disease selected from the group consisting of an autoimmune disease, inflammation and neural disease without affecting a living body adversely when administered in an effective amount to a mammal including a human i.e. multiple sclerosis. The autoimmune disease, inflammation and neural disease is a multiple sclerosis. Thus, a pharmaceutical agent containing hyaluronan as an active ingredient i.e. the tetrasaccharide according to claim 1 has a therapeutic effect and a prophylactic effect against multiple sclerosis.

Also, a pharmaceutical agent which contains hyaluronan as an active ingredient, i.e. the tetrasaccharide according to claim 1, can inhibit reduction in cell viability and/or can activate a cell without affecting a living body adversely when administered in an effective amount to a mammal including a human.

The pharmaceutical agent contains hyaluronan as an active ingredient, i.e. the tetrasaccharide according to claim 1, and can inhibit the expression of certain activated cytokine- and chemokine-associated genes without affecting a living body adversely when administered in an effective amount to a mammal including a human.

The pharmaceutical agent can be formulated into a desired dosage form as it is or in combination with a carrier, excipient and other additives as desired for forming a pharmaceutical product for intravaginal administration

An oral formulation may for example be a solid formulation such as a powder, granule, capsule, and tablet; a liquid formulation such as a syrup, elixir, and emulsion. A powder formulation can be obtained as a mixture with an excipient such as lactose, starch, crystalline cellulose, calcium lactate, calcium hydrogen phosphate, magnesium aluminate metasilicate, and silicic anhydride. A granule formulation can be obtained by means of a wet or dry granulation process with adding, in addition to the excipients listed above, a binder such as a sugar, hydroxypropyl cellulose, polyvinyl pyrrolidone and the like, a binder such as a carboxymethyl cellulose, and calcium carboxymethyl cellulose, and a disintegrant such as a carboxymethyl cellulose, and calcium carboxymethyl cellulose, as desired. A tablet formulation can be obtained by compacting the powder or the granule described above as it is or together with a lubricant such as magnesium stearate, and talc. The powder or the granule described above can be coated with an enteric coating base such as hydroxypropyl methyl cellulose phthalate, methyl methacrylate copolymer and the like, or may be coated with ethyl cellulose, carnauba wax, and hydrogenated oil, whereby formulating into an enteric or sustained-release formulation. A hard capsule formulation can be obtained by filling the powder or the granule described above as in a hard capsule. A soft capsule formulation can be obtained by mixing hyaluronan or its salt with a glycerin, polyethylene glycol, sesame oil, olive oil and the like followed by coating with a gelatin membrane. A syrup formulation can be obtained by dissolving a sweetener such as a sugar, sorbitol, and glycerin together with hyaluronan or its salt in water. In addition to a sweetener and water, an essential oil or ethanol may be added to form an elixir, or a gum arabic, tragacanth, polysorbate 80 or sodium carboxymethyl cellulose may be added to form an emulsion or suspension. Such a liquid formulation may be supplemented also with a flavor, colorant, preservative and the like, if desired.

A parenteral formulation may for example be an injection formulation, rectal formulation, pessary, dermal application formulation, inhalant, aerosol, instillation formulation and the like. An injection formulation can be obtained by adding to hyaluronan or its salt a pH modifier such as hydrochloric acid, sodium hydroxide,lactic acid,sodiumlactate,sodium monohydrogen phosphate, and sodium dihydrogen phosphate; an osmotic agent such as sodium chloride, and glucose; and a distilledwater for injection, followed by a sterile filtration, and then filling into an ampoule. In addition, it may be supplemented also with mannitol, dextrin, cyclodextrin, gelatin and the like, and lyophilized under vacuum to form an injection formulation for reconstitution before use. It can also be formulated into an emulsion for injection by adding to hyaluronan or its salt an emulsifier such as lecithin, polysorbate 80, and polyoxyethylene hydrogenated castor oil followed by emulsifying in water.

A rectal formulation can be obtained by adding to hyaluronan or its salt a suppository base such as a mono-, di- or triglyceride of a cocoa butter fatty acid, and polyethylene glycol, followed by warming to melt, and then casting into a mold and cooling, or by mixing hyaluronan or its salt with a polyethylene glycol, soybean oil and the like followed by coating with a gelatin membrane. A dermal application formulation can be obtained by adding to hyaluronan or its salt a white petrolatum, beeswax, liquidparaffin, polyethylene glycol and the like if necessary with warming and then kneading. A tape formulation can be obtained by kneading hyaluronan or its salt together with an adhesive such as rosin, and alkyl acrylate polymer, followed by spreading over an unwoven fabric and the like. An inhalant can be obtained by dissolving or dispersing hyaluronan or its salt in a propellant such as a pharmaceutically acceptable inert gas followed by filling into a pressure-resistant container.

### (Administration mode)

The administration mode of a pharmaceutical agent of the presentinvention containing hyaluronan as an active ingredient i.e. the tetrasaccharide according to claim 1 is intraspinal, administration.

### (Dosage)

While the dosage may appropriately be selected depending on the disease to be subjected, age, general condition and body weight of the patient and the like, it is generally 0.05 to 50 mg/kg which is given once a day or in divided doses.

### (Toxicity)

Hyaluronan employed in the invention i.e. the tetrasaccharide according to claim 1 exhibited almost or completely no cytotoxicity at a dose exhibiting a biological activity of a pharmaceutical.

A pharmaceutical agent according to the invention is further detailed below with reference to Examples, which are not intended to restrict the technological scope of the invention.

### [Example 1]

In this example, HA4 was administered to an experimental autoimmune encephalomyelitis (EAE) which is a multiple sclerosis model to examine its efficacy.

Four-week old Lewis rats for multiple sclerosis models were purchased and used when they became five-week old. In accordance with the method by Shibaki et al (Shibaki K, Nomura K, Ono R, Shimazu K, Inhibition of experimental autoimmune encephalomyelitis by NINJINEIYOTO, SHINKEICHIRYO 19(2): 159-166, 2002), 300 µg/animal of a guinea pig myelin basic protein (GPMBP, Sigma) was dissolved in 50 µl of PBS, which was then supplemented with an equivalent amount of Freund Complete Adjuvant (FCA, Difco) and sterilized Mycobacterium tuberculosis (MT, Difco) at the concentration of 0.75mg/ml, each 50 µl of which was inoculated to each paw of both rear extremities of the animal.

In this example, the multiple sclerosis model animals were received HA4 immediately after the inoculation or upon the onset of neural symptoms.

### Administration of test substances

In this example, HA4 was prepared at 1 mg/ml and 10 mg/ml. Specifically, HA4 was prepared by the method of Tawada et al. (Tawada A, Masa T, Oonuki Y, Watanabe A, Matsuzaki Y, Asari A. Large-scale preparation, purification, and characterization of hyaluronan oligosaccharides from 4-mers to 52-mers. Glycobiology, 2002; 12(7): 421-6). As a control, physiological saline was used.

At the two time points, that is, immediately after the inoculation and upon the onset of the disease as confirmed by the observation of neural symptoms, a catheter was placed in a medullary space of the multiple sclerosis model animal, where an intradural administration was effected during a predetermined period. For a continuous administration, an osmotic pump (model 2004, Alzet) was employed. The animals were assigned to the treatment groups shown in Table 1.

**Table 1**

| Group | Test substance | Dose (µg/day) | Dosing concentration (µg/ml) | Start of dosing | Treatment period | Number of animals |
|---|---|---|---|---|---|---|
| 1 | Physiological saline | - | - | Immediately after inoculation of antigen | 22 Days | 6 |
| 2 | HA4 | 6 | 1 | Immediately after inoculation of antigen | 22 Days | 6 |
| 3 | HA4 | 6 | 1 | Time upon onset* | 11 Days | 4 |
| 4 | HA4 | 60 | 10 | Time upon onset* | Single dose | 4 |

| | | | | | | |
|---|---|---|---|---|---|---|
| *: Time point of observation of EAE grade 1 (reduced tonus of tail). | | | | | | |

### EAE neural symptom evaluation

Everyday after the antigen inoculation, the neural symptoms were assessed by two observation personnel with one of the scores of the following 5 grades.
EAE grade:
0: No symptoms
1: Loss of tail tone
2: Hind limb weakness
3: Hind limb paralysis sometimes accompanied with incontinence of urine and feces
4: Hind limb and fore limb paralysis

### Results

### 1) Effect (prophylactic) of intraspinal continuous administration of HA4 immediately after inoculation (challenge) and thereafter

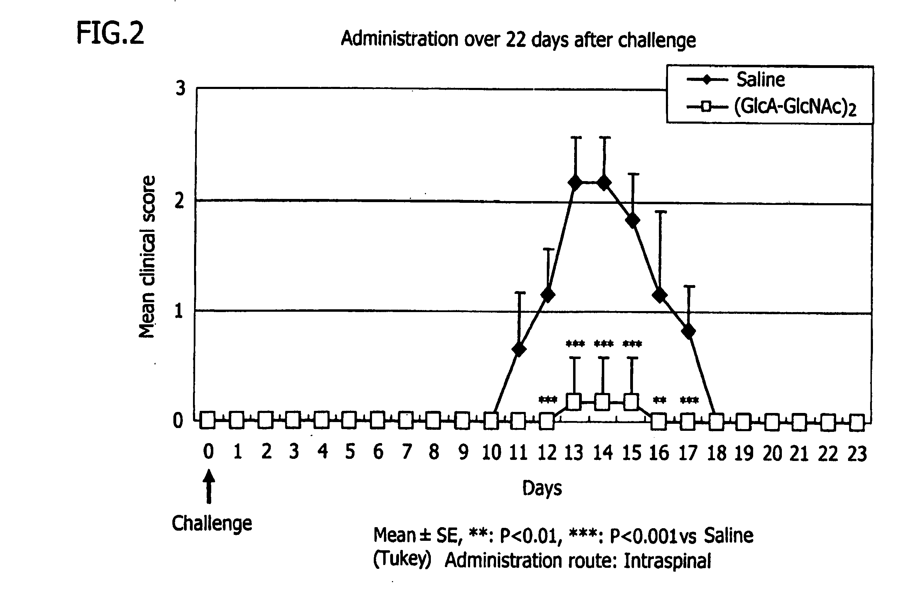

After the inoculation of the antigen, HA4 intraspinal continuous administration made the neural symptoms milder clearly comparing with the physiological saline (FIG. 2). FIG. 2 shows a graph obtained by plotting the average of a score of the EAE neural symptom described above and the days after the inoculation on the ordinate. When comparing the neural symptoms at the EAE climax, the clinical score in the physiological saline group on Day 13 after the antigen inoculation was 2.2 ± 0.41, while that in the HA4 continuous administration group on Day 13 was 0.2 ± 0.41 which was significantly lower (p < 0.001), with only 1/6 of the cases developing the disease in the HA4 continuous administration group. Based on the results shown in FIG. 2, it was proven that the pharmaceutical agent containing the hyaluronan as an active ingredient is effective for the prophylaxis of the multiple sclerosis.

### 2) Effect (therapeutic) of intraspinal continuous administration of HA4 immediately after onset of disease

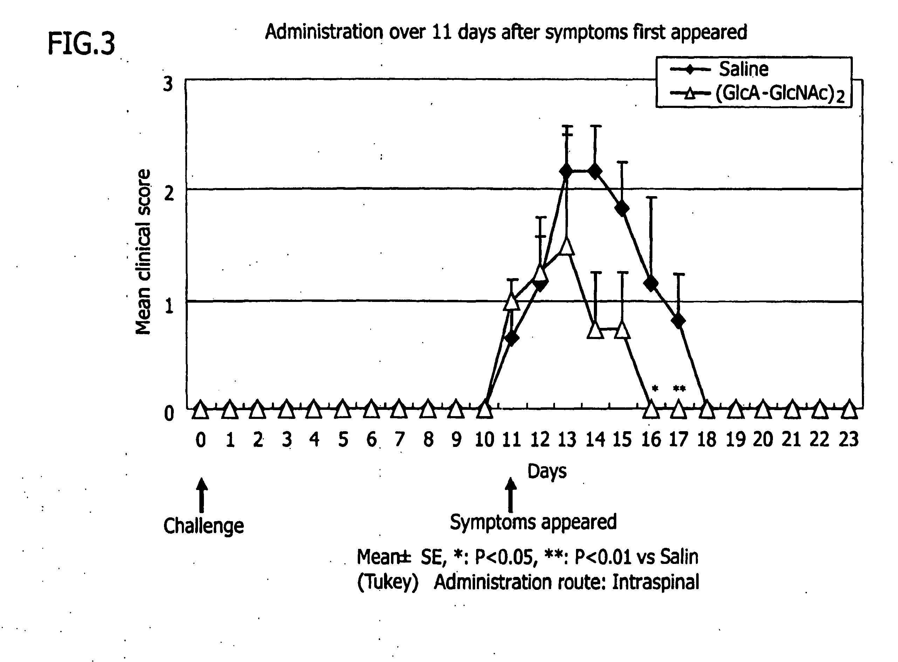

After the onset of the disease, the HA4 intraspinal continuous administration caused the neural symptoms which became milder clearly when comparing with the physiological saline group (FIG. 3) . FIG. 3 shows a graph obtained by plotting the average of a score of the EAE neural symptom described above and the days after the inoculation on the ordinate. When comparing the neural symptoms at the EAE climax, the clinical score in the physiological saline group on Day 13 after the antigen inoculation was 2.2 ± 0.41, while that in the HA4 continuous administration group on Day 13 was 1.5 ± 1.0 which was significantly lower. When comparing the diseased period, 6.5 ± 0.55 days in the physiological saline group and 4 . 3 ± 1.5 days in the (glucNac-GlcA)₂ continuous treatment group revealed a significant reduction (p < 0.01) in the latter. Based on the results shown in FIG. 3, it was proven that the pharmaceutical agent containing HA4 as an active ingredient is effective for the prophylaxis of the multiple sclerosis.

### 3) Effect (therapeutic) of intraspinal single administration of HA4 immediately after onset of disease.

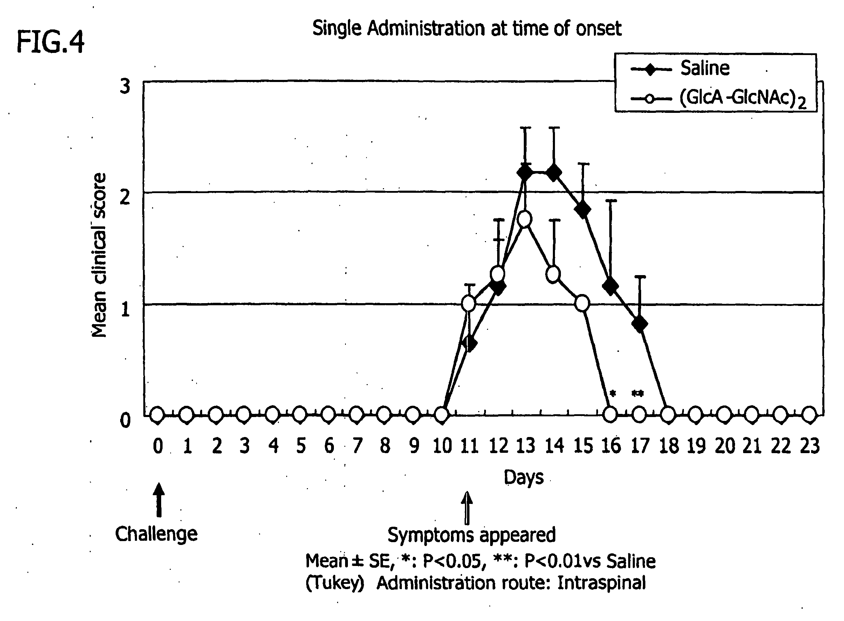

After the onset of the disease, the HA4 intraspinal single administration made the neural symptoms milder clearly comparing with the physiological saline group (FIG. 4). FIG. 4 shows a graph obtained by plotting an average of a score of the EAE neural symptom described above and the days after the inoculation on the ordinate. When comparing the neural symptoms at the EAE climax, the clinical score in the physiological saline group on Day 13 after the antigen inoculation was 2.2 ± 0.41, while that in the HA4 continuous administration group on Day 13 was 1.8 ± 0.5 which was significantly lower. When comparing the diseased period, 6.5 ± 0.55 days in the physiological saline group and 5.0 ± 1.5 days in the HA4 continuous treatment group revealed a significant reduction (p < 0.001) in the latter. Based on the results shown in FIG. 4, it was proven that the pharmaceutical agent containing HA4 as an active ingredient is effective for the prophylaxis of the multiple sclerosis.

### [Example 2]

In this example, effect of the inventive pharmaceutical agent on cell viability was measured using Rhodamine 123. Rhodamine 123 exhibits a fluorescence whose intensity is increased in a manner dependent on the membrane potential of a mitochondria which acts pivotally in an energy metabolism. Accordingly, the degree of the staining with Rhodamine 123 serves as an index of the mitochondrial activity, thus the index of the cellular activity (see, non-patent reference 2).

### Cell to be activated

In this example, a K562 (referred to as human erythroleukemia cell or human erythroblastoid leukemia cell) was used. The K562 was purchased from RIKEN, Japan.

In this example relating to preparation of test substance, HA4 was prepared at 100 ng/ml. Specifically, HA4 was prepared by the method of Tawada et al. (Tawada A, Masa T, Oonuki Y, Watanabe A, Matsuzaki Y, Asari A. Large-scale preparation, purification and characterization of hyaluronan oligosaccharides from 4-mers to 52-mers. Glycobiology, 2002; 12(7):421-6) and the concentration was adjusted using a physiological saline.

### Experimental method

First, the K562 was incubated under the condition described below. The culture medium for the K562 was an RPMI-1640 medium. In this example, the K562 was incubated in Groups 1 to 3 shown below. Each group was cultured under the condition described below. The culture medium in Group 3 was supplemented with HA4 (100 ng/ml).
Group 1: 80 minutes at 37°C
Group 2: 20 minutes at 43°C (heat treatment) followed by 60 minutes at 37°C
Group 3: 20 minutes at 43°C (heat treatment) followed by 60 minutes at 37°C

After the incubation, a Rhodamine 123 dissolved in an MI medium was added to each group. The final concentration of the Rhodamine 123 was 1 µg/ml. After adding the Rhodamine 123 followed by incubation at 37°C for 10 minutes, the K562 was washed with the RPMI medium.

The K562 after washing was inoculated at 1×10⁴ cells/ml in a 96-well plate and a photograph was taken using a fluorescent microscope (Nikon) . The image was sent to anAdobe Photoshop (Adobe Systems) where the fluorescent intensity of a cell was measured.

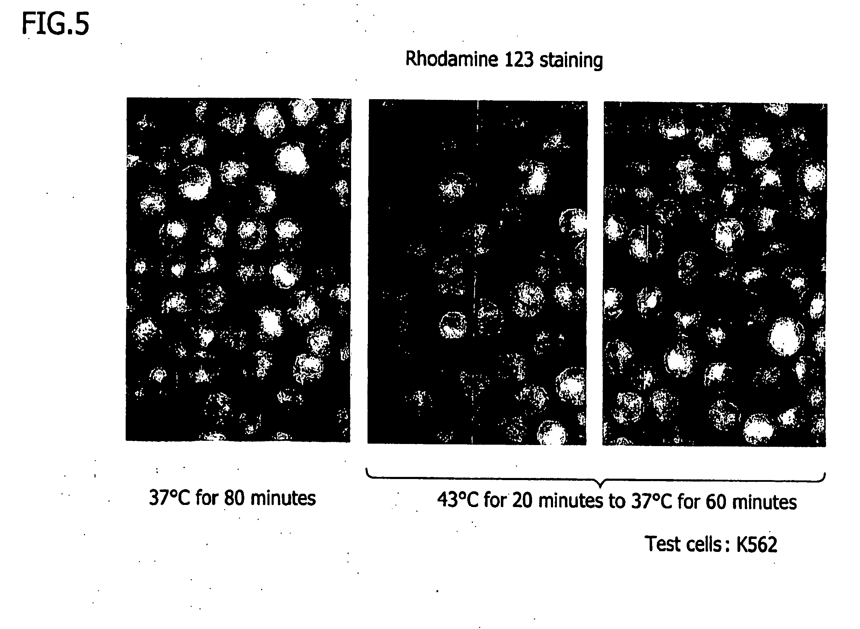

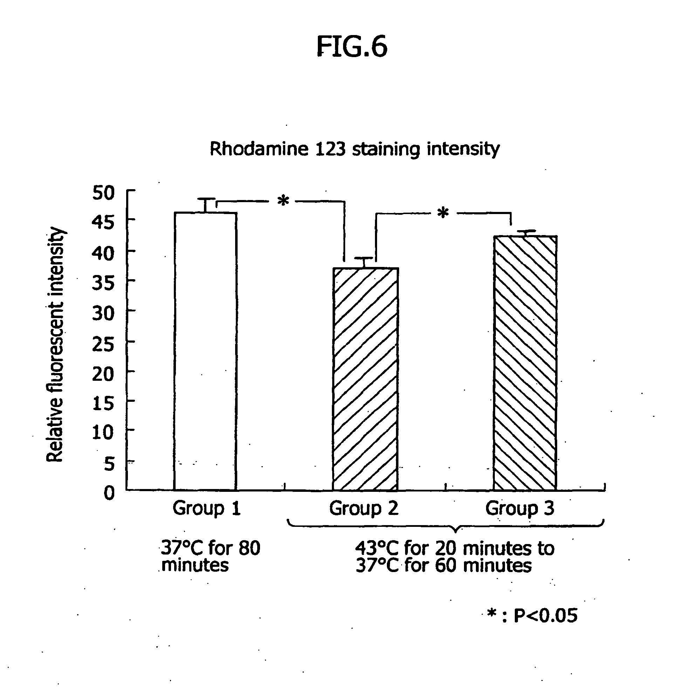

The image of each group is shown in FIG. 5, and the measured fluorescent intensity is shown in FIG. 6. Based on the results shown in FIGS. 5 and 6, the heat treatment at 43°C for 20 minutes caused a reduction in the Rhodamine 123 staining performance (Group 2), while the presence of HA4 inhibited such a reduction (Group 3). The difference in the fluorescent intensity between Groups 2 and 3 was significant.

### Discussion

Apparent from the results described above, HA4 inhibited the reduction in the mitochondrial membrane potential, that is, the reduction in the mitochondrial activity. These findings suggest that HA4 has an effect to suppress the reduction in the mitochondrial activity which is inevitable under hazardous condition (heat treatment), or has an effect to recover the mitochondrial activity which has once been reduced, thus has a mitochondria activating effect. Since the mitochondria is an organelle which produces a cellular energy (ATP), HA4 has a cell viability enhancing effect.

### REFERENCES

1.Martin W, Hoffmeisterher M, Rotte C, Henze K. An overview of endosymbiotic models for the origins of eukaryotes, their ATP-producing organelles (mitochondria and hydrogenosomes), and their heterotrophic lifestyle. Biol Chem. 2001 Nov; 382(11): 1521-39.
2. Hatefi Y. ATP synthesis in mitochondria. Eur J Biochem. 1993 Dec 15; 218(3): 759-67.

### [Example 3]

In this example, the cell viability enhancement of a pharmaceutical agent comprising the tetrasaccharide according to claim 1 was assessed using a DNA chip capable of monitoring a gene expression promotion/inhibition.

### Experimental method

First, the K562 was incubated in Groups 1 and 2 in the RP plate medium described above. The both groups were incubated at 42°C for 20 minutes followed by 37°C for 30 minutes. The medium of Group 2 was supplemented with HA4 (10 ng/ml).

After the incubation, the medium was removed by centrifugation at 1000 rpm. The obtained cells were stored in a deep freezer at -60°C. From the cells thus stored, RNA was extracted according to a standard method. The extracted RNA was subjected to the DNA chip to analyze gene expressions. The DNA chip gene expression analysis was subtracted to DNA CHIP Research Inc. Specifically, the trade name: AceGene Human Oligo Chip 30K 1 Chip Version manufactured by DNACHIP Research Inc. was employed.

### Results

The results of the DNA chip expression analysis revealed that the cells incubated in the medium containing HA4 exhibited a significant change in the expression profile of many genes involved in the cell viability listed in Table 2.

**Table 2: HA4 cell viability enhancing effect**

| | HAA+/- ratio | Functions |
|---|---|---|
| <Apoptosis-related> | | |
| STK17b (DARK2) | 0.09 | Signal inducing apoptosis |
| pawr (Par-4) | 0.45 | Increased expression in neuron being ready for apoptosis |
| Caspase 2 | 0.27 | TNF-induced apoptosis executing factor |
| Granzyme H | 0.38 | Serine protease Apoptosis inducing |

| <Transcription control-related> | | |
|---|---|---|
| DNAJ2 | 0.34 | Heat-inducibletranscriptional repressor (Transcription inhibition) |
| TAF9L | 0.43 | Transcription factor (Transcription inhibition) |

| <Heat shock protein-related> | | |
|---|---|---|
| dnaj (hsp40) homolog | 2.62 | Heat shock protein |

As shown in Table 2, HA4 served (1) to inhibit the apoptosis-related gene expressions, (2) to inhibit the transcription inhibition-related gene expression and (3) to promote the heat shock protein-related gene expression. Specifically, HA4 inhibited the gene expression of STK17b (DDRAK2), pawr (Par-4), Caspase 2 and Granzyme H, which are factors relating to the apoptosis induction or execution. In addition, HA4 inhibited the gene expression of DNAJ2 and TAF9L which are factors causing a transcription inhibition. Moreover, HA4 promoted the gene expression of dnaj (hsp40) homolog which is a heat shock protein.

### Discussion

Since STK17b (Dmm) and pawr (Par4) among the apoptosis-related genes in the results shown above are the factors serving to induce or promote the apoptosis, the inhibition of the expression of these genes leads to the inhibition of the apoptosis. For STK17b (DRAK2), see Sanjo H, Kawai T, Akira S. DRAKs, novel serine/threonine kinases related to death-associated protein kinase that trigger apoptosis. J Biol Chem. 1998 273 (44) : 29066-71 . For pawr (Par-4), see Johnstone RW, See RE, Sells SF, Wang J, Muthukkumar S, Englert C, Haber DA, Licht JD, Sugrue SP, Roberis T. Rangnekar VM, Shi Y. A novel repressor, par-4, modulates transcription and growth suppression functions of the Wilms' tumor suppressor WT1 . Mol Cell Biol. 1996 16 (12): 6945-56 and Mattson MP, Duan W, Chan SL, Camandola S. Par-4: emarerg pivotal player in neuronal apoptosis and neurodegenerative disorders. J Mol Neurosci. 1999 Aug-Oct; 13(1-2): 17-30.

Also since Caspase 2 is an apoptosis executing factor, the relevant gene expression inhibition leads to an inhibition of apoptosis. For Caspase 2, see ZhivotovskyB, Orrenius S. Caspase-2 functionin response to DNA damage. Biochem Biophys Res Commun. 2005 331(3): 859-67.

Since Granzyme H is a factor by which a lymphocyte induces the apoptosis in other cells, the relevant gene expression inhibition leads to an inhibition of apoptosis. For Granzyme H, see Sedelies KA, Sayers TJ, Edwards KM, Chen W, Pellicci DG, Godfrey DI, Trapani JA Discordant regulation of granzyme H and granzyme B expression in human lymphocytes. J Biol Chem, 2004 279(25): 26581-7. Epub 2004 Apr 6.

The results described above indicated that the inhibition of the gene expression of DNAJ2 and TAFgL leads to the recovery of the transcription activity once having been inhibited. For DNAJ2, see Terada K, Mori H. Human DnaJ homologs dj2 and dj3, and bag-1 are positive cochaperones of hsc70. J Biol Chem, 2000 275 (32) : 24728-34. For TAFgL, see Chen Z, Manley Jl, In vivo function a analysis of the histone 3-like TAF9L and a TAF9-related factor, TAF9L. J Biol Chem. 2003 278(37): 35172-83.

We have already reported that HA4 has a heat shock protein 72 (Hsp72) expression promoting effect (Xu H, Ito T, Tawada A, Maeda H, H, Yamanokuchi H, Isahara K, Yoshida K, Uchiyama Y, Asari A. Effect of hyaluronan oligosaccharides on the expression of heat shock protein 72. J Biol Chem, 2002 10; 277 (19) : 17308-14). In this example, an analysis using a DNA chip revealed that HA4 promotes the gene expression of dnaj (hsp40) homolog which is a heat shock protein. The dnaj (hsp40) homolog has a intracellular protein denaturation inhibiting effect and a cell death inhibiting effect, similarly to Hsp72.

Based on the results of this example discussed above, HA4 was revealed to have functions such as the apoptosis inhibition, transcription activity recovery and protein denaturation inhibition. Since these functions are all related to the cell viability, it can be concluded that HA4 has a cell viability enhancing effect.

### [Example 4]

In this example, the cytokine-associated gene and chemokine-associated gene expression inhibition of a pharmaceutical agent comprising the tetrasaccharide according to claim 1 was assessed using a DNA chip capable of monitoring a gene expression promotion/inhibition.

Experimental method: First, the K562 was incubated in Groups 1 and 2 in the RPMI medium described above. The both groups were incubated at 42°C for 20 minutes followed by 37°C for 30 minutes. The medium of Group 2 was supplemented with HA4 (10 ng/ml).

After the incubation, the medium was removed by centrifugation at 1000 rpm. The obtained cells were stored in a deep freezer at -60°C. From the cells thus stored, RNA was extracted according to a standard method. The extracted RNA was subjected to the DNA chip to analyze the gene expression. The DNA chip gene expression analysis was subtracted to DNA CHIP Research Inc. Specifically, the trade name: AceGene Human Oligo Chip 30K 1 Chip Version manufactured by DNA CHIP Research Inc. was employed.

### Results

The results of the DNA chip expression analysis revealed that the cells incubated in the medium containing HA4 exhibited a significant change in the expression profile of many genes involved in the cell viability listed in Table 3.

**Table 3**

| | HA4(+)/HA4(-) ratio | Functions |
|---|---|---|
| IFN-γ | 0.11 | Th1-type cytokine |
| Mig(CXCL9) | 0.11 | Th1-type C-X-C chemokine |
| IL-5 | 0.28 | Th2-type cytokine |
| IL-17b | 0.31 | Th1-type cytokine |
| IL-18RAP | 0.32 | Bound to IL-li8 to aid for receptor binding |
| CCL28 | 0.32 | Chemokine (epithelium, produced by KC) |
| IL-1β | 0.36 | Inflammatory cytokine |
| IFN-ω1 | 0.5 | NK cell activating cytokine |

As shown in Table 3, the HA4 treatment resulted in a plurality of inhibitions of the cytokine-associated gene and chemokine-associated gene expression. Among the cytokine-associated genes and chemokine-associated genes shown in Table 3, for IFN-γ gene, see Schroder K, Hertzog PJ, Ravasi T, Home DA. Interferon-gamma: an overview of signals, mechanisms and functions. J Leukoc Biol. 2004 75 (2) : 163-89. For Mig (CXCL9) gene, see Farber JM. Mig and IP-10: CXC chemokines that target lymphocytes. J Leukoc Biol. 1997 61(3): 246-57. For IL-5 gene, see Adachi T, Alam R. The mechanism of IL-5 signal transduction Am J Physiol. 1998 275(3 Pt 1) : C623-33. For IL-17b, see Li H, Chen J, Huang A, Stinson J, Heldens S, Foster J, Dowd P, Gurney AL, Wood WI. Cloning and characterization of IL-17B and IL-17C, two new members of the IL-17 cytokine family. Proc Nat1 Acad Sci USA. 2000 18 97 (2) : 773-8. For IL-18RAP, see Cheung H, Chen NJ, Cao Z, Ono N, Ohashi PS, Yeh WC . Accessory protein-like is essential for IL-18-mediated signaling. J Immunol. 2005 174 (9) : 5351-7. For CCL28, see Wang W, Soto H, Oldham ER, Buchanan ME, Homey B, Catron D, Jenkins N, Copeland NG, Gilbert DJ, Nguyen N, Abrams J, Kershenovich D, Smith K, McClanahan T, Vicari AP, Zlotnik A. Identification of a novel chemokine (CCL28), which binds CCR10 (GPR2). J Biol Chem. 2000 275 (29): 22313-23. For IL-1β, see Okamura H. IL-1 family (IL-laipha/beta, IL-iRa, IL-18), IL-16, IL-17. Nippon Rinsho. 2005 63 Supp-1 4: 226-33. For IFN-ω1, see Bekisz J, Schmeisser H, Hernandez J, Goldman ND, Zoon KC. Human interferons alpha, beta and omega. Growth Factors. 2004 22(4): 243-51. And Adolf GK Maurer-Fogy I, Kalsner I, Cantell K. Purification and characterization of natural human interferon omega 1. Two alternative cleavage sites for the signal peptidase. J Biol Chem. 1990 265(16): 9290-5.

### Discussion

In this example, the HA4 treatment resulted in the inhibition of the cytokine-associated gene and chemokine-associated gene expression. Since the K562 cells employed here were leukocyte-derived cells, it naturally undergoes the expression of the cytokine-associated gene and chemokine-associated gene.

Since HA4 promotes Hsp72 expression (see, Xu II, I to T, Tawada A, Maeda H, Yamanokuchi H, Isahara K, Yoshida K, Uchiyama Y, Asari A. Effect of hyaluronan oligosacharides on the expression of heat shock protein 72, J Biol. Chem, 2002 10, 277(19): 17300-14), it is possible that Hsp72 is recognized in vivo by γδT cells to produce an IL-10 and then the IL-10 inhibits the production of various inflammatory cytokines and chemokines. However, since the γδT cells are not included in this example, the expression of the cytokine-associated genes and chemokine-associated genes involved in an inflammation or autoimmune disease is inhibited directly by the HA4 treatment.

### [Example 6]

By a DNA array analysis using a K562 cell, HA4 was proven to inhibit the production of various cytokines such as IL-1β and IFNγ in the presence of a heat shock. In this example, an U937 cell known to produce various cytokines via a LPS stimulation was used to examine HA4 for its effect on the cytokine production.

### Materials

### 3. Test substance HA4, prepared in accordance with the method of Tawada et al. (1).

2. Cell: U937 cell (human monocyte line), purchased from Dainipon Sumitomo Seiyaku.
3. Culture medium: RPMI medium (containing 10% FBS).
4. LPS E.coli, 0111B4, Chemicon.

### Methods

An U937 cell was disseminated in a 2 ml aliquot in a 6-well microplate at 5×10⁵ cell/ml, supplemented with an LPS at a final concentration of 100 or 1000 ng/ml, and then incubated for 24 hours with 5% CO₂ at 37°C in the presence or absence of HA4 (100 ng/ml) . The culture supernatant was centrifuged at 3000 rpm for 5 minutes to obtain a test substance. A 1 ml aliquot of the recovered supernatant was subjected to Human Cytokine Antibody array (Raybio) using an ELISA (ENDOGEN) to detect the production of various cytokines.

More specifically, the following procedure was employed to measure the production of the cytokines by the cytokine array and the ELISA.

### Cytokine antibody array

A 2 ml aliquot of a blocking buffer was added to a membrane blotted with an antibody, which was then shaken for 30 minutes. The blocking buffer was removed, and a 1 ml aliquot of the culture supernatant was added and shaken at room temperature for 2 hours, washed 5 times, and then admixed with a primary antibody solution. After agitating at room temperature for 1.5 hours followed by washing 5 times, a secondary antibody solution was added and agitated overnight at 4°C. After washing 5 times, a chemiluminescence was allowed to develop and photographed by a Polaroid. The photograph was scanned to obtain its digital data which were subjected to an Image J to measure the luminescent intensity. The ratio based on the luminescent intensity of an internal standard placed in 6 positions in the membrane was calculated. In addition, a relative level (%) based on the expression level in a non-treatment group (NT) being 100 was calculated.

### ELISA (IL-6)

To a microplate, 50 µl of a biotinylated antibody solution and 50 µl of the culture supernatant were added, and allowed to stand at room temperature for 2 hours. After washing with a washing buffer three times, 100 µl of a streptoavidin-HRP solution was added, allowed to stand at room temperature for 30 minutes, and washed with the washing buffer three times. A TMB was added, allowed to stand for at room temperature 30 minutes, and then a quencher was added to stop the reaction and the absorbance at 450 nm was measured (reference: 562 nm). The IL-6 concentration (pg/ml) was calculated based on the standard solutions measured in parallel.

### Results

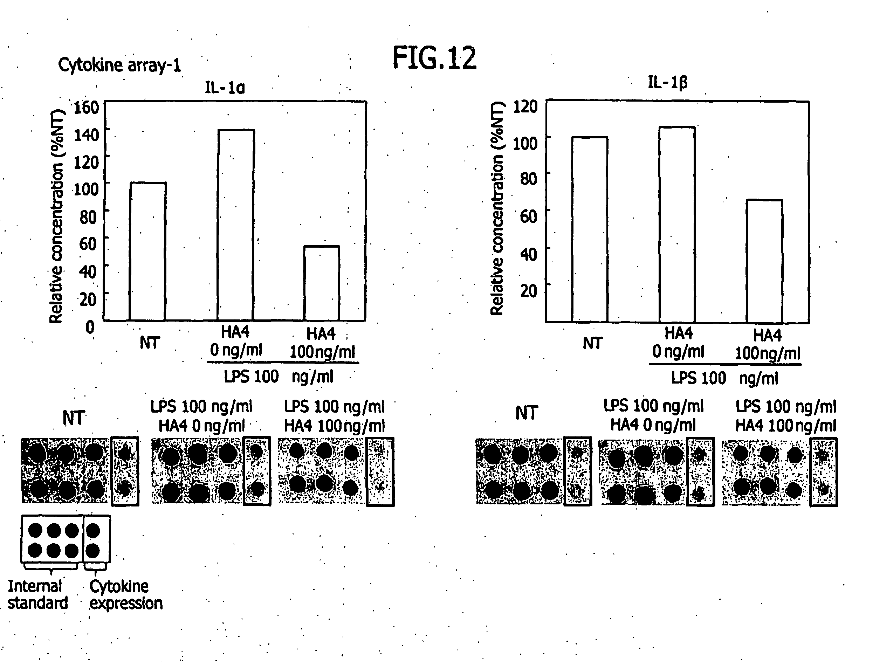

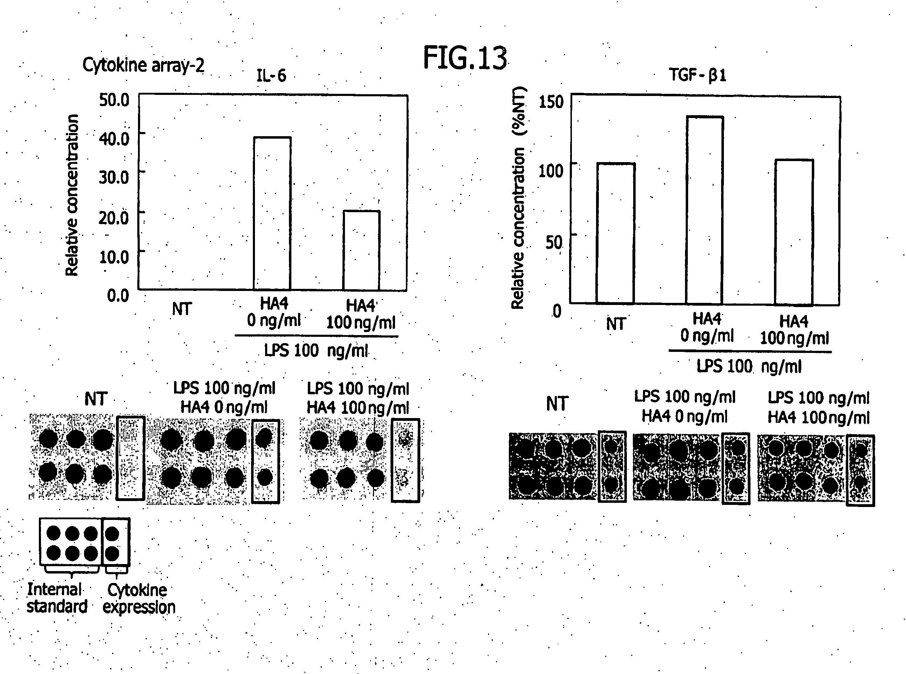

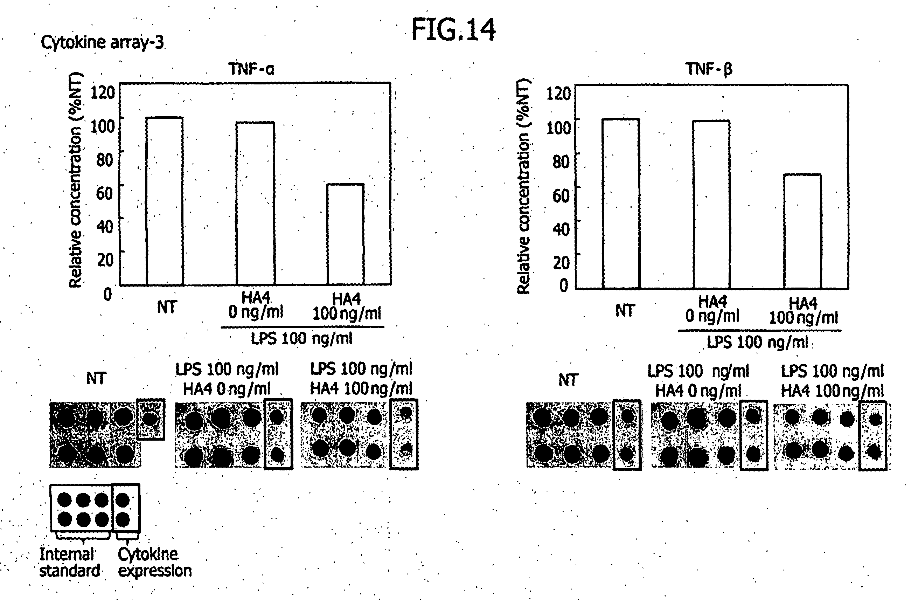

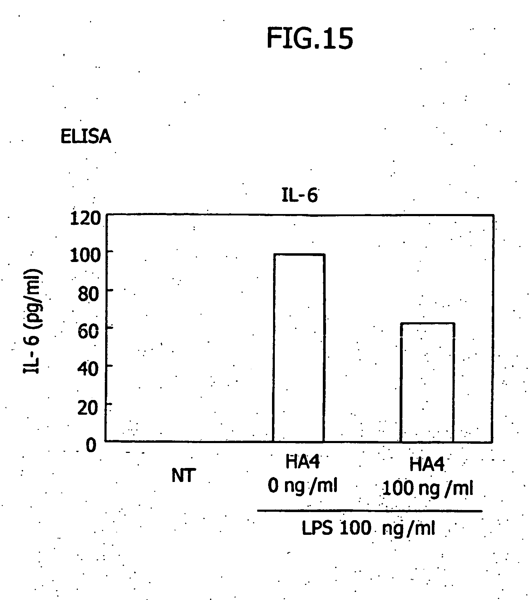

In the presence of a stimulation with 100 ng/ml of the LPS, the addition of HA4 at 100 ng/ml resulted in a reduction in the production of inflammatory cytokines IL-1α, β, IL-6, TGF-β, TNF-α and β (FIGS. 12 to 14 (cytokine array), FIG. 15 (ELISA)). In the graphs shown in FIGS. 12 to 14, the ordinate represents a relative value (%) based on the expression level in the non-treatment group (NT) being 100. Only the IL-6 was represented as a relative concentration.

These results revealed that HA4 has a pharmacological effect as an inflammatory cytokine production inhibitor.

### REFERENCES

1. Tawada A, Masa T, Oonuki Y, Watanabe A, Matsuzaki Y, Asari A. Large-scale preparation, purification, and characterization of hyaluronan oligosaccharides from 4-mers to 52-mers. Glycobiology. 2002 12(7): 421-6.

### [Example 7]

HA4 administered intrathecally after an antigen inoculation (challenge) to an experimental autoimmune encephalomyelitis (EAE) which is a multiple sclerosis model leads to a significant inhibition of the development of a neural symptom such as a paralysis. On the other hand, a reduced expression of the transthyretin is known to result in an increased noradrenaline expression. The noradrenaline has an inflammatory cytokine expression inhibiting effect and an activity promoting effect. Based on such an understanding, the inhibition of the expression of the neural symptom such as the paralysis by HA4 is considered to involve the reduction in the expression of the transthyretin. In the Example 1, the effect of HA4 on the autoimmune disease/inflammation and the multiple sclerosis which is a neural disease was described. Accordingly, in this example, the experimental autoimmune encephalomyelitis (EAE) model which is a multiple sclerosis model was treated with HA4 or a physiological saline (negative control), and on Day 14 after the treatment when the symptom became severest, the brain and spinal cord tissues were collected and subjected to a DNA array to examine the action mechanisms.

### <Materials and methods>

Lewis rats which were four-week old when purchased and five-week old when used were employed as experimental animals. As a test substance, HA4 (1 mg/ml, 10 mg/ml) was employed. HA4 was prepared by the method of Tawada et al. (Tawada A, Masa T, Oonuki Y, Watanabe A, Matsuzaki Y, Asari A. Large-scale preparation, purification and characterization of hyaluronan oligosaccharides from 4-mers to 52-mers. Glycobiology, 2002; 12(7): 421-6).

### Preparation of multiple sclerosis model (EAE)

(1) Test substances
   * Guinea pig myelin basic protein (GPMBP), Sigma
   * Sterilized Mycobacterium tuberculosis (MT, Difco)
   * Freund's adjuvant Complete (FCA, Difco)
   * Physiological saline (PS)
(2) Model preparation

In accordance with the method by Shibaki et al (Shibaki K, Nomura K, Ono R, Shimazu K, Inhibition of experimental autoimmune encephalomyelitis by NINJIN-EIYOTO, SHINKEI-CHIRYO 19(2): 159-166, 2002), 300 µg/animal of the GPMBP was dissolved in 50 µl of PBS, which was then supplemented with an equivalent amount of FCA and sterilized Mycobacterium tuberculosis adjusted at the concentration of 0.75 mg/ml, each 50 µl of which was inoculated to each paw of both rear extremities of the animal.

Immediately after the antigen inoculation, a catheter was placed in a medullary space, where an intrathecal administration was effected during a predetermined period. For a continuous administration, an osmotic pump (model 2004, Alzet) was employed. The animals were assigned to two groups shown below and one group was treated with HA and the other with the physiological saline as a control.

**Table 5**

| Group | Test substance | Dose (µg/day) | Dosing concentration (mg/ml) | Start of dosing | Treatment period | Number of animals |
|---|---|---|---|---|---|---|
| 1 | Physiological saline | - | - | Immediately after inoculation of antigen | 14 Days | 6 |
| 2 | HA | 6 | 1 | Immediately after inoculation of antigen | 14 Days | 6 |

### DNA array analysis

On Day 14 after administration, a cerebrospinal tissue was taken and pooled for each group, and then subjected to the RNA extraction. The RNA sample thus extracted was subjected to a DNA array analysis by TAKARABIO. The results are shown in the following table.

**Table 6**

| | Expression level ratio (HA/physiological saline) |
|---|---|
| Transthyretin | 0.38 |
| Fibroblast growth factor receptor substrate 2 | 2.3 |
| Decoy TRAIL receptor without death domain | 2.0 |

### <Discussion>

A reduced transthyretin expression is known to lead to an increased noradrenaline expression (Caggiula M, Batocchi AP, Frisullo G, Angelucci F, PatanellaAK, Sancricca C, Nociti V, Tonali PA, Mirabella M. Neurotrophic factors and clinical recovery in relapsing-remitting multiple sclerosis. Scand J Immunol. 2005 Aug; 62 (2) : 176-82). The noradrenaline has an inflammatory cytokine expression inhibiting effect (Sousa JC,Grandela C, Fernandez-Ruiz J, de Miguel R, de Sousa L, Magalhaes AI, Saraiva MJ, Sousa N, Palha JA. Transthyretin is involved in depression-like behaviour and exploratory activity. J Neurochem. 2004; 88 (5) : 1052-8) and a searching behavior/activity increasing effect (Feinstein DL, Heneka MT, Gavrilyuk V, Dello Russo C, Weinberg G, Galea E. Noradrenergic regulation of inflammatory gene expression in brain. Neurochem Int. 2002; 41(5): 357-65. Review). From the understanding described above, the inhibitory effect of HA4 on the ultromotivity reduction/paralysis is considered to involve the transthyretin expression reduction described above.

A fibroblast growth factor receptor substrate 2 is a receptor substrate for a fibroblast growth factor (GFG) and for a neural growth factor such as a nerve growth factor. Accordingly, an increase in the fibroblast growth factor receptor substrate 2 means an increase in the sensitivity to the neural growth factor expressed in the multiple sclerosis (Caggiula M, Batocchi AP, Frisullo G, Angelucci F, Patanella AK, Sancricca C, Nociti V, Tonali PA, Mirabella M. Neurotrophic factors and clinical recovery in relapsing-remittingmultiple sclerosis. Scand J Immunol. 2005Aug; 62 (2) : 176-82; TriacaV, Tirassa P, Aloe L. Presence of nerve growth factor and TrkA expression in the SVZ of EAE rats: evidence for a possible functional significance. Exp Neurol. 2005; 191(1): 53-64. ; Laudiero LB, Aloe L, Levi-Montalcini R, Buttinelli C, Schilter D, Gillessen S, Otten U. Multiple sclerosis patients express increased levels of beta-nerve growth factor in cerebrospinal fluid. Neurosci Lett. 1992 Nov 23; 147(1): 9-12). Accordingly, it can be assumed that the HA4 treatment provides the effects of the neural growth factors, such as neuron death inhibition, neuron differentiation and axonal growth by which the symptoms of the multiple sclerosis were suppressed (Villoslada P, Genain CP. Role of nerve growth factor and other trophic factors in brain inflammation. ProgBrainRes. 2004; 146: 403-14. Review. ; Gielen A, Khademi M, Muhallab S, Olsson T, Piehl F. Increased brain-derived neurotrophic factor expression in white blood cells of relapsing-remitting multiple sclerosis patients. Scand J Immunol. 2003; 57(5): 493-7. ; Villoslada P, Hauser SL, Bartke I, Unger J, Heald N, Rosenberg D, Cheung SW, Mobley WC, Fisher S, Genain CP. Human nerve growth factor protects common marmosets against autoimmune encephalomyelitis by switching the balance of T helper cell type 1 and 2 cytokines within the central nervous system. J Exp Med. 2000 15; 191 (10) : 1799-806. ; Boutros T, Croze E, Yong VW. Interferon-beta is a potent promoter of nerve growth factor production by astrocytes. J Neurochem. 1997; 69-(3): 939-46. ; Massaro AR, Soranzo C, Bigon E, Battiston S, Morandi A, Carnevale A, Callegaro L. Nerve growth factor (NGF) in cerebrospinal fluid (CSF) from patients with various neurological disorders. Ital J Neurol Sci. 1994; 15(2): 105-8. ; Althaus HH, Kloppner S, Schmidt-Schultz T, Schwartz P. Nerve growth factor induces proliferation and enhances fiber regeneration in oligodendrocytes isolated from adult pig brain; Neurosci Lett. 1992 3; 135(2): 219-23).

A decoy TRAIL receptor without death domain exhibits a competitive inhibition of the binding of the TRAIL to its receptor (Pan G, Ni J, Wei YF, Yu G, Gentz R, Dixit VM. An antagonist decoy receptor and a death domain-containing receptor for TRAIL. Science 1997 8; 277 (5327): 815-8). Accordingly, HA4 is considered to inhibit the cell death of neurons and an oligodendrocyte (myelin) by the TRAIL via an increase in the decoy TRAIL receptor without death domain (urewicz A, Matysiak M, Andrzejak 5, Selmaj K. TRAIL-induced death of human adult oligoclendrocytes is mediated by JNK pathway. Glia. 2006 15; 53(2): 158-66).

### Review

### <Effects of HA4 on multiple sclerosis, spinal cord injury and asthma/allergic disease>

As described above, when HA4 was given to the rat EAE (experimental allergic encephalomyelitis) model which is the multiple sclerosis model, the inhibition of the neural symptoms was observed.

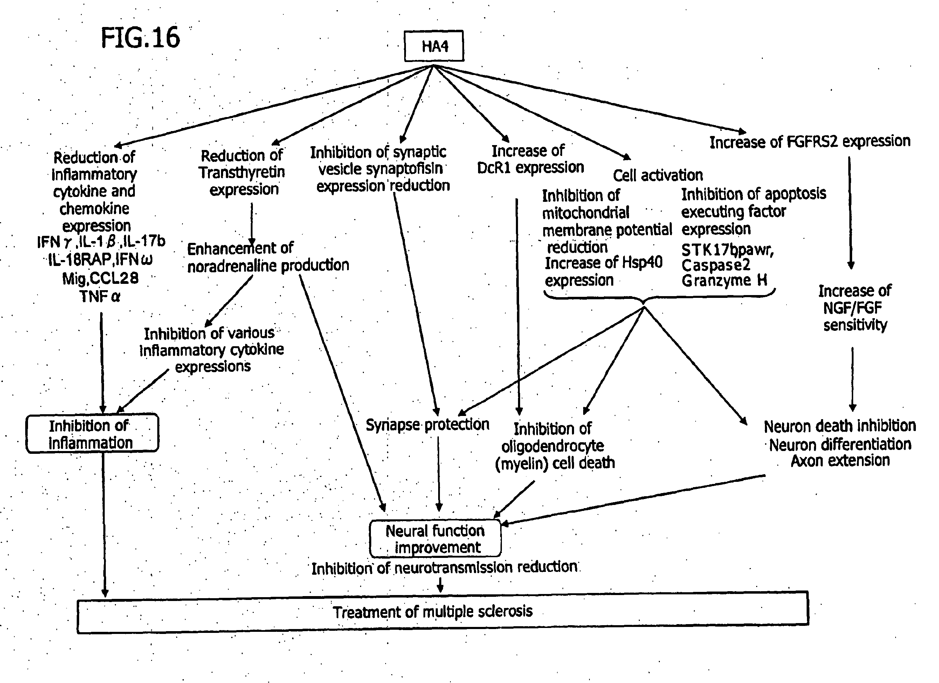

Also based on the DNA array analysis using the rat cerebrospinal tissue, the mitochondrial potential activity and DNA array analysis using the K562 cells , HA4 was revealed to have (1) an inflammation inhibiting effect, and (2) a neural function improving effect (inhibition of a reduction in a neurotransmission) via a, oligodendrocyte (myelin) cell death inhibiting effect and neuron death inhibition/neuron differentiation/axon extension (FIG. 16).

In the DNA array analysis using the K562 cells, the inhibition of the IL-1β expression was observed. The IL-1β is known to be a factor which exacerbates a spinal cord injury (Yang L, Jones NR, Blumbergs PC, Van Den Heuvel C, Moore EJ, Manavis J, Sarvestani GT, Ghabriel MN. Severity-dependent expression of pro-inflammatory cytokines in traumatic spinal cord injury in the rat. JClinNeurosci. 2005Apr; 12 (3) : 276-84) . Theabovementioned HA4 effects (1) and (2) also contributes to the inhibition of the exacerbation/treatment in the spinal cord injury. The fact discussed above indicates a therapeutic effect of HA4 also on the spinal cord injury.

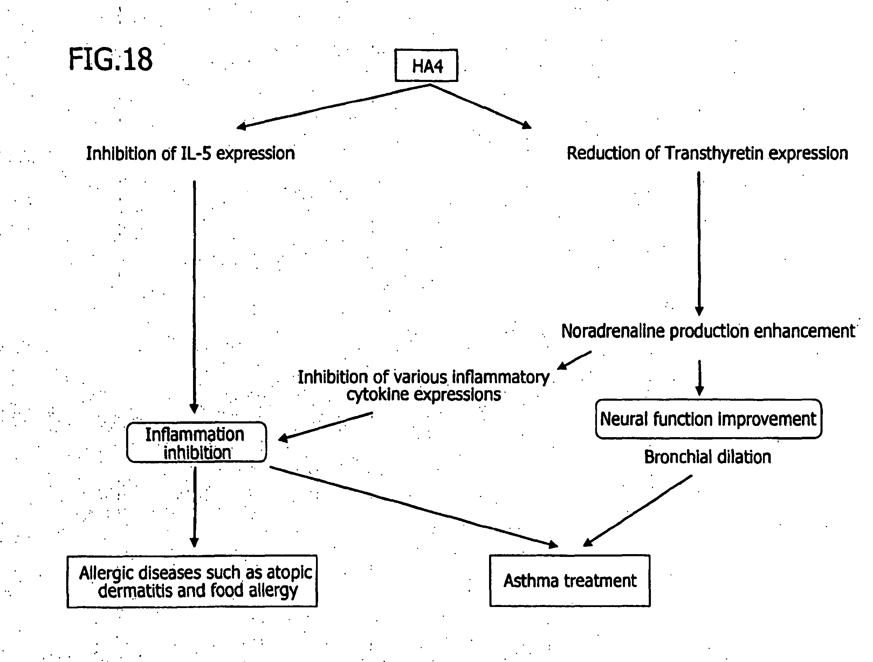

The DNA array analysis using the K562 cells showed the inhibition of the IL-5 expression. The IL-5 is known to be a factor which exacerbates an allergic disease such as an asthma (Hamelmann E, Gelfand EW). IL-5-induced airway eosinophilia the key to asthma? Immunol Rev. 2001 Feb; 179: 182-91). In addition, the transthyretin reducing effect of HA4 contributes to the inhibition of the exacerbation/treatment in the cases of asthma or allergic diseases, since it leads to an increased noradrenaline production and a bronchial dilation. The fact discussed above indicates a therapeutic effect of HA4 also on the asthma and allergic diseases (FIG. 18).

## Claims

1. A tetrasaccharide containing 2 units, with a single unit being -D-glucuronic acid-β-1,3-D-N-acetylglucosamine-β-1,4-, for use in the treatment or prevention of a multiple sclerosis via intraspinal administration.

2. The tetrasaccharide according to claim 1, **characterized in that** is administered in a dosage of from 0.05 to 50 mg/kg body weight of the patient.

3. The tetrasaccharide according to claim 2, **characterized in that** it is administered once a day.

4. The tetrasaccharide according to any of claims 1 to 3, **characterized in that** it is administered as a sterile filtrated injection formulation containing a pH modifier, an osmotic agent and distilled water for injection, provided in an ampoule.

5. The tetrasaccharide according to claim 4, **characterized in that** the pH modifier is selected from the group consisting of hydrochloric acid, sodium hydroxide, lactic acid, sodium lactate, sodium monohydrogen phosphate and sodium dihydrogen phosphate.

6. The tetrasaccharide according to claim 4 or 5, **characterized in that** the osmotic agent is sodium chloride or glucose.

7. The tetrasaccharide according to any of claims 4 to 6, **characterized in that** the injection formulation is supplemented also with mannitol, dextrin, cyclodextrin or gelatin.

8. The tetrasaccharide according to any of claims 1 to 3, **characterized in that** it is administered as an emulsion for injection containing an emulsifier and water.

9. The tetrasaccharide according to a claim 8, **characterized in that** the emulsifier is selected from the group consisting of lecithin, polysorbate 80, and polyethylene hydrogenated castor oil.

## Patentansprüche

1. Tetrasaccharid, das zwei Einheiten aufweist, wobei es sich bei einer Einheit um -D-Glukuronsäure-β-1,3-D-N-Acetylglucosamin-β-1,4- zur Verwendung bei der Behandlung oder Prävention von Multiple Sklerose über intraspinale Verabreichung handelt.

2. Tetrasaccharid nach Anspruch 1, **dadurch gekennzeichnet, dass** es in einer Dosierung von 0,05 bis 50 mg/kg Körpergewicht des Patienten verabreicht wird.

3. Tetrasaccharid nach Anspruch 2, **dadurch gekennzeichnet, dass** es einmal täglich verabreicht wird.

4. Tetrasaccharid nach einem der Ansprüche 1 bis 3, **dadurch gekennzeichnet, dass** es als eine sterile, gefilterte Injektionszusammensetzung verabreicht wird, welche einen pH-Modifizierer, ein osmotisches Mittel und destilliertes Wasser für die Injektion aufweist, bereitgestellt in einer Ampulle.

5. Tetrasaccharid nach Anspruch 4, **dadurch gekennzeichnet, dass** der pH-Modifizierer aus der Gruppe ausgewählt wird, die folgendes umfasst: Chlorwasserstoffsäure, Natriumhydroxid, Milchsäure, Natriumlactat, Natrium-Monohydrogenphosphat und NatriumDihydrogenphosphat.

6. Tetrasaccharid nach Anspruch 4 oder 5, **dadurch gekennzeichnet, dass** es sich bei dem osmotischen Mittel um Natriumchlorid oder Glucose handelt.

7. Tetrasaccharid nach einem der Ansprüche 4 bis 6, **dadurch gekennzeichnet, dass** die Injektionszusammensetzung auch mit Mannitrol, Dextrin, Cyclodextrin oder Gelatine ergänzt wird.

8. Tetrasaccharid nach einem der Ansprüche 1 bis 3, **dadurch gekennzeichnet, dass** es als eine Emulsion für eine Injektion mit einem Emulgator und Wasser verabreicht wird.

9. Tetrasaccharid nach Anspruch 8, **dadurch gekennzeichnet, dass** der Emulgator aus der Gruppe ausgewählt wird, die folgendes umfasst: Lecithin, Polysorbat 80 und mit Polyethylen hydriertes Rizinusöl.

## Revendications

1. Tétrasaccharide contenant 2 fragments, avec un seul fragment qui est de l'acide-β-1,3-D-N-acétylglucosamine-β-1,4- -D-glucuronique, destiné à être utilisé dans le traitement ou la prévention d'une sclérose en plaques par le biais d'une administration intraspinale.

2. Tétrasaccharide selon la revendication 1, **caractérisé en ce qu'**il est administré dans un dosage allant de 0,05 à 50 mg/kg de poids corporel du patient.

3. Tétrasaccharide selon la revendication 2, **caractérisé en ce qu'**il est administré une fois par jour.

4. Tétrasaccharide selon l'une quelconque des revendications 1 à 3, **caractérisé en ce qu'**il est administré en tant que formulation pour injection filtrée stérile contenant un modificateur de pH, un agent osmotique et de l'eau distillée pour l'injection, fournie dans une ampoule.

5. Tétrasaccharide selon la revendication 4, **caractérisé en ce que** le modificateur de pH est sélectionné parmi le groupe constitué de l'acide chlorhydrique, l'hydroxyde de sodium, l'acide lactique, le lactate de sodium, le monohydrogénophosphate de sodium et le dihydrogénophosphate de sodium.

6. Tétrasaccharide selon la revendication 4 ou 5, **caractérisé en ce que** l'agent osmotique est du chlorure de sodium ou du glucose.

7. Tétrasaccharide selon l'une quelconque des revendications 4 à 6, **caractérisé en ce que** la formulation pour injection est également additionnée de mannitol, dextrine, cyclodextrine ou gélatine.

8. Tétrasaccharide selon l'une quelconque des revendications 1 à 3, **caractérisé en ce qu'**il est administré en tant qu'émulsion pour injection contenant un émulsifiant et de l'eau.

9. Tétrasaccharide selon la revendication 8, **caractérisé en ce que** l'émulsifiant est sélectionné parmi le groupe constitué de la lécithine, du polysorbate 80 et de l'huile de ricin hydrogénée avec du polyéthylène.
